# EUROPEAN PATENT APPLICATION

(11) **EP 4 488 353 A1**
(43) Date of publication of application: **08.01.2025**
(21) Application number: 23306125.8
(22) Date of filing: 04.07.2023
(51) Int. Cl.: C12F 3/06, C12G 1/08, C12N 1/20

(54) **PROCESSING AND USE OF WHITE WINE LEES TO PROMOTE MALOLACTIC FERMENTATION**

(71) Applicant: Université de Bordeaux, 33000 Bordeaux (FR); Institut Polytechnique de Bordeaux, 33402 Talence Cedex (FR); Institut national de recherche pour l'agriculture, l'alimentation et l'environnement, 75338 Paris Cedex 07 (FR); École Nationale Supérieure des Sciences Agronomiques de Bordeaux Aquitaine Bordeaux Sciences Agro, 33175 Gradignan Cedex (FR); Universitat Rovira I Virgili, 43003 Tarragona (ES)
(72) Inventor: NIOI, Claudia, 33170 Gradignan (FR); BALMASEDA RUBINA, Aitor, 01003 Vitoria-Gasteiz (ES); LUCAS, Patrick, 33650 Martillac (FR); MIOT-SERTIER, Cécile, 33370 Yvrac (FR)
(74) Representative: Novagraaf Technologies

(57) **Abstract**

The present invention relates to a new method to promote malolactic fermentation (MLF) and/or growth of lactic acid bacteria in food products, in particular in red wines, using dried white wine lees.

## Description

### Technical field of the invention

The present invention relates to a new method to promote malolactic fermentation (MLF), in particular in red wines, using dried white wine lees.

In the description below, references in square brackets ([ ]) refer to the list of references at the end of the text.

### State of the art

Malolactic fermentation (MLF) consists in the transformation of L-malic acid into L-lactic acid by lactic acid bacteria (LAB). It is a key stage in the vinification of red and some white wines and is fundamental to the quality of wines. It usually occurs after alcoholic fermentation (AF, transformation of sugars into alcohol by yeasts), when LAB - mainly the species *Oenococcus oeni* - manage to proliferate and reach a high population. During MLF, the metabolism of the bacteria induces a deacidification of the wine, which improves its taste, microbial stability, aromatic complexity. It is compulsory and included in the specifications of most red wine appellations.

MLF usually occurs spontaneously when the indigenous LAB, which originate from the cellar or the surface of the grapes, have developed sufficiently. However, wine is a hostile environment for microorganisms because of its acidity (very low pH), high alcohol content (> 12 %), the presence of polyphenols, sulphites and other inhibitors that slow down bacterial growth. In addition, after alcoholic fermentation, the wine is depleted of sugars and nutrients that the yeasts have consumed. The medium is therefore very difficult for bacteria. However, the bacteria can take advantage of the autolysis of the yeasts that occurs at the end of alcoholic fermentation, which releases molecular compounds that enrich the medium in nutrients, thus, enhancing bacterial growth. Nevertheless, bacterial growth and MLF do not always occur immediately after alcoholic fermentation, but sometimes after several weeks or months, and sometimes never. One explanation could be the presence of the inhibitors mentioned above (polyphenols), or a lack of nutrients. This last parameter seems to be very important when analyzing the situations of MLF difficulties during the vinification of red wines. Indeed, after alcoholic fermentation, it is common to proceed to a "devatting", which consists of separating the "clear wine" contained in the liquid part of the tanks from the "press wine" which is obtained by pressing the solid part composed of the grape skins, uncrushed berries and yeast lees. This press wine is much richer in nutrients than the free-run wine, and always achieves MLF more easily.

MLF is a stage of winemaking that is feared by winemakers because of its "capricious" nature. If it does not occur quickly, it can lead to delays in marketing, difficulties in storing wines or losses in quality. To remedy this, winemakers can use commercial LAB, which are industrially produced and inoculated into the wines to start MLF. This solution solves MLF difficulties in almost all cases. However, it presents a significant cost that is prohibitive for the majority of winemakers. Moreover, it does not correspond to the wish of most of them, which is to use "indigenous" bacteria to carry out MLF. Another solution is to improve the development of indigenous bacteria by using commercial products rich in nitrogen and vitamins useful for the development of lactic bacteria, that are produced from industrially produced inactivated yeast extracts. However, these products are expensive and are not always effective enough to help achieve MLF.

The vinification of wine generates various by-products such as stalks, pomace, seeds and lees. All of these by-products have attracted the interest of researchers for their valorization in different sectors, notably human food, human health and animal feed. This biomass is rich in interesting compounds. The polyphenolic fraction is the most studied, in particular for its antioxidant properties and targeted applications in various sectors (Devesa-Rey R. et al. 2011; Ruiz-Moreno M. Jet al., 2015; Yammine S., et al., 2019) [1-3]. Grape pomace (60 % of wine-making by-products) is the most widely valorized. Wine lees is the second, (25 %) of by-products produced. Although the quantity of lees produced is very large, they are not widely used, especially lees from white wine vinification, due to their low content of polyphenolic compounds. Until now, one of the uses of lees is as an additive in the distillation process for certain spirits, such as grappa, to increase the alcoholic potential of the raw material to be distilled. Recent scientific studies have shown the value of using wine lees to obtain proteins with functional activity (De Iseppi A., et al., 2020; De Iseppi et al., 2021.) [4-5] and antioxidant capacity (Nioi, 2022) [6]. At present, no enhancement of lees as a nutrient for microorganisms is proposed.

Therefore, there is still a need to find an effective, simple, quick and inexpensive solution to overcome the problem of "difficult" MLF, in particular in red wines.

### Description of the invention

In order to solve the problem of "difficult" MLF, the inventors had the idea of using a nutrient mixture derived from lees in order to promote the growth of LAB and significantly reduce the duration of MLF,. The nutrient mixture is obtained by using white wine lees, which are a by-product of white wine production.

For this purpose, white wine lees are dried by an easy process, resulting in a final product containing 95 % dry matter. This drying rate ensures that the nutritional properties of the nutrient blend are preserved, as well as the absence of microbial contamination. The nutrient blend obtained from these lees has significantly better nutritional properties than the existing commercial nutrients. Moreover, it is obtained at a low cost and in a virtuous process of valorization of a by-product of winemaking.

The use of white wine lees has aroused the interest of the inventors, first because of the amounts available, and secondly because of their composition, which is different from that of other by-products, leading to the prospect of new applications. White wine lees are composed of the yeast matrix (from alcoholic fermentation) and plant debris from the grapes ("bourbes"). The composition of white wine lees remains relatively unexplored and undetailed, but studies report the presence of proteins, peptides, polysaccharides, mannoproteins and lipids (Fornairon-Bonnefond, C. et al., 2001) [7]. It is known that some of these compounds can play a role of interest as microbial growth stimulators (Bustos et al., 2004a, 2004b; Balmaseda et al., 2021a; Balmaseda et al., 2021b; Balmaseda, et al, 2022) [8-12].

Therefore, the inventors have studied the potential of white wine lees to act as promoters of bacterial growth and MLF achievementin red winemaking. Although the main application of these white wine lees is oenological, it is also considered to use them as bacterial nutrients in various applications such as food and drink productions: dairy products (yoghurts, cheeses), fermented vegetables (olives, gherkins, sauerkraut), fermented alcoholic drinks (wine, beer, cider), cold meats (ham, sausages) or sourdough bread.

An object of the present invention is therefore a method of inducing in a food product the conversion of L-malic acid to L-lactic acid and/or the growth of lactic acid bacteria, said method comprising the following steps:
a) recovery of white wine lees after alcoholic fermentation and before wine ageing;
b) drying of white wine lees from step a);
c) addition of dried white wine lees from step b) to the food product;
d) keeping of the food product from step c) under conditions which allow conversion of L-malic acid to L-lactic acid;
e) recovery of the fermented (MLF) food product.

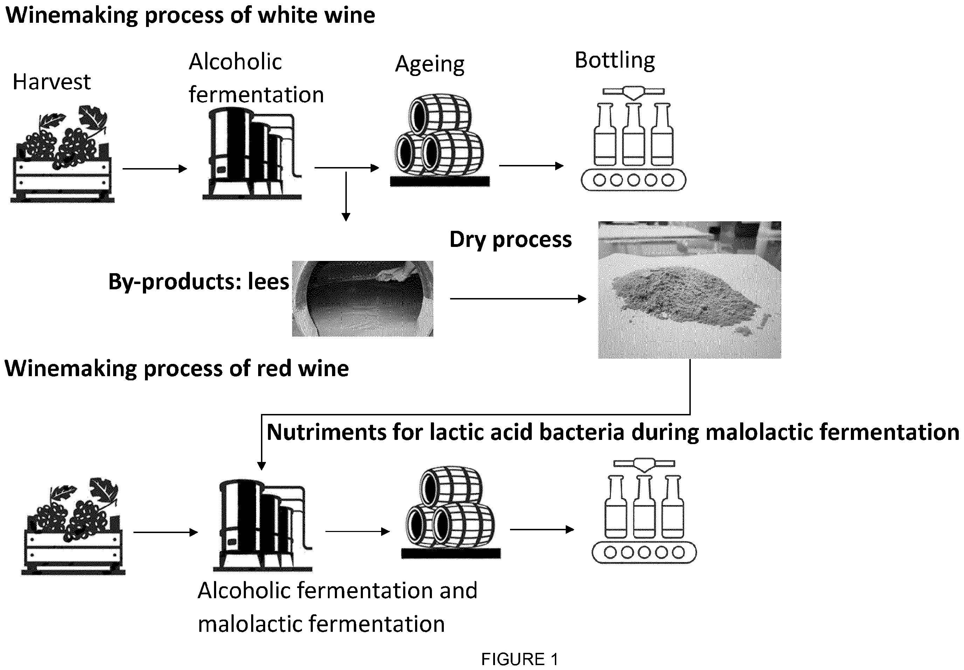

The claimed method is illustrated in Figure 1.

According to a particular embodiment of the method of the present invention, the drying in step b) is carried out by atomisation or freeze-drying.

According to a particular embodiment of the method of the present invention, the dried white wine lees contain from 80 % to 95 % of dry matter, preferably from 90 to 95% of dry matter, more preferably 95% of dry matter.

According to a particular embodiment of the method of the present invention, the addition in step c) is carried out at a concentration of 0.1 to 1 g/L, preferably of 0.3 to 1 g/L, more preferably of 0.5 to 0.3 g/L, of dried white wine lees.

Another object of the present invention is also the use of dried white wine lees to promote malolactic fermentation in a food product, in particular in fermented beverages like wines, ciders, etc.... More generally, the white wine lees can promote LAB growth in other fermented food or for LAB production at industrial scale for different applications.

According to a particular embodiment of the present invention, the food product is chosen from the group consisting of fermented alcoholic drinks (e.g. wine, cider), fruit juices, dairy products, fermented vegetables, cold meats, sourdough bread.

According to a particular embodiment of the present invention, the fermented alcoholic drinks are chosen from the group consisting of red wines, beers and ciders. In this case, the addition in step c) is carried out during alcoholic fermentation, at the end of alcoholic fermentation, or after alcoholic fermentation and before ageing.

Another object of the present invention is a powdered nutrient comprising or consisting of dried white wine lees, and eventually at least one oenological acceptable excipient. Said dried white wine lees can be obtained by atomisation or freeze-drying. Said dried white wine lees contain from 80 % to 95 % of dry matter, preferably from 90 to 95% of dry matter, more preferably 95% of dry matter.

### Brief description of the figures

Figure 1 represents experimental method: white wine vinification, recovery of lees and re-use in red wine vinification as MLF activators.
Figure 2 represents the use of lees as a source of nutrients in a culture medium containing 100 mL/L red grape juice. A. Growth curves of *Oenococcus oeni* strains VF, VP41, IOEB-SARCO 277 and IOEB-SARCO 450 at 25°C; (●) without lees, or in the presence of 10 g/L of (■) lees 1, or (▲) lees 3.
Figure 3 represents the correlation between MLF duration and amount of lees added (0, 0.25, or 0.5 g/L) for O. *oeni* strains VF (●), VP41 (■), 450 (▲). The tests were carried out in synthetic wine by triplicate. The correlation equation for each strain is also provided next to each regression line.
Figure 4 represents the evolution of the L-malic acid consumption during MLF in wines non inoculated (spontaneous) or inoculated with O. *oeni* VF at 10⁴, 10⁶ CFU/mL in presence of wine lees collected after FA (●) and three months after (■). The tests were performed in red wine. Dotted lines and full lines correspond respectively to 0.25 g/L and 0.5 g/L of added lees. The grey line represents the control without no lees addition.
Figure 5 represents the growth of *Brettanomyces bruxellensis* CRBO L0611, CRBO L0422, CRBO L0424 in two different red wines (A, B) (●) without lees, or in the presence of 0.5 g/L of (■) lees 1, or (▲) lees 4.
Figure 6 represents malic acid degradation kinetics of MALOBOOST^{®}. Study of a Cabernet Sauvignon wine recalcitrant to MLF (TAV: 14.04% vol.; pH: 3.62; L-malic acid: 3.34 g/L). Comparison of MLF with and without seeding with selected lactic bacteria at 1 g/hL. Addition of MALOBOOST^{®} (30 g/hL) 24 hours before the addition of bacteria for the inoculated modality and at the same time for the uninoculated wine. Test temperature: 18°C.

### EXAMPLES

### EXAMPLE 1 : MATERIALS AND METHODS

### 1. Bacterial and yeast strains and growth conditions

Four commercial *Oenococcus oeni* strains were used in this study: VF (Vitilactic F, Martin Vialatte, Magenta, France), VP41 (Lalvin VP41, Lallemand Inc., Canada), IOEB-SARCO 277 (SB3, Laffort, Bordeaux, France), and IOEB-SARCO 450 (Lactoenos 450 Preac, Laffort, Bordeaux, France). Three *Brettanomyces bruxellensis* strains from CRBO collection were used: CRBO L0611, CRBO L0422 and CRBO L0424.

O. *oeni* strains were grown in liquid grape juice medium (1 L): 250 mL of red grape juice, 5 g of yeast extract, 1 mL of Tween 80, adjusted to pH 4.8. The cultures were incubated at 25°C for around 5 days until late exponential phase. Bacterial enumeration was performed by epifluorescence microscopy for inoculation, and by plating in solid grape juice medium (20 g/L agar, supplemented with 100 mg/L of pimaricin) during the experiments. For inoculation in red wine, commercial freeze-dried O. *oeni* VF was used according to manufacturer's instructions.

*8. bruxellensis* strains were grown on YPG plates (1 L): 10 g of yeast extract, 10 g of peptone, 20 g of glucose and 25 g of agar. Each strain was gradually adapted to wines with successive subcultures.

### 2. Lees collection, preparation, and characterization

Wine lees (lees 1, 2, and 3) come from Chateau Giscours (CDO Medoc, Labarde, France) and were collected during 2021 vintage. Another wine lees sample (lees 4) come from Château Couhins (CDO Pessac-Leognan, France) after alcoholic fermentation of vintage 2022. Lees 1 and 2 correspond to a white wine vinification of Viognier grapes inoculated with S. *cerevisiae* Zymaflore CH9 (Laffort, Bordeaux, France). Lees 1 were collected immediately after alcoholic fermentation completion, and lees 2 were collected after 3 months of ageing in barrel. Lees 3 were collected after alcoholic fermentation from a Chardonnay white wine fermented with the same strain. Lees 4 have the same fermentative origin as lees 3, coming collected from a different winery and vintage.

All lees were freeze-dried (FreeZone 4.5 plus, Labconco) and stored at 4 °C in a hermetically closed jar until experiments.

### Microbiological analysis of lees

Also, the microbial composition of the lees was analyzed. 100 mg of each wine lees were resuspended in 1 mL of saline solution. Then, 100 µL of each suspension was spread out on grape juice agar and YPG (20 g/L glucose, 10 g/L yeast extract, 10 g/L peptone) agar plates. After 2-10 days, isolated colonies were counted and identified by MALDI-TOF mass spectrometry using *MALDI* Biotyper^{®} (MBT) from Bruker and homemade database.

### 3. Biomass production

The ability of malolactic starter species (*O*. *oeni*) to develop in a wine byproduct low-cost medium was tested. To this aim grape juice medium containing 100 mL/L of red grape juice was supplemented with non, 2.5, 5, or 10 g/L of lees 1 or lees 3. In all cases pH was adjusted to 4.8. 10 mL screwed vials (Fisher Scientific, Hampton, New Hampshire, USA ref: 11981523) were filled with 9 mL of the obtained media and inoculated with *O. oeni* VF for a population of 10⁴ - 10⁵ cell/mL. Vials were closed wi th screw cap-magnetic (Agilent Technologies, hdsp cap 18 mm PTFE/sil 100 pk, Les Ulis, France). Caps were perforated by two hypodermic needles: one for allowing CO₂ release (0.8 × 38 mm, Terumo, Tokyo, Japan), and the other (0.6 × 80 mm, B. Braun, Melsungen, Germany) connected to a 2 mL syringe (B. Braun, Melsungen, Germany) for allowing sampling. Fermentations were incubated statically at 25 °C for two weeks. Bacterial population was enumerated each day by plating. Each condition was tested in triplicate.

The suitability of this medium (100 mL/L red grape juice, 10 g/L of lees 1 and lees 3 at pH 4.8) was tested with the other three *O. oeni* strains - VP41, IOB-SARCO 277, IOEB-SARCO 450.

9 mL of medium were inoculated with each *O. oeni* strain for a population of 10⁵ cell/mL into the same vial system. Each condition was incubated statically at 25 °C for two weeks. Besides, the effect of the temperature and pH was evaluated. For comparison, the same medium was evaluated at 20 °C or adjusting the pH to 3.5. Each condition was tested in triplicate.

### 4. Malolactic fermentation

### 4.1. Malolactic fermentation performance evaluation

First, a screening was performed with wine lees 1 and *O. oeni* VF in a synthetic wine (wine like medium, WLM). WLM was prepared following method described by (Balmaseda et al., 2023) [13]. *O. oeni* VF was inoculated for a population of 10², 10⁴, or 10⁶ cell/mL in presence of 0, 0.25, or 0.5 g/L of wine lees 1. Inoculated WLM was transferred to a 10 mL syringe (BD, Franklin Lakes, USA) coupled with a hypodermic needle (0.8 × 38 mm, Terumo, Tokyo, Japan) and incubated statically at 20 °C. Each condition was performed in triplicate. Bacterial population and L-malic acid concentration was monitored periodically. L-Malic Acid Assay Kit (Megazyme, Wicklow, Ireland) was used for L-malic acid quantification.

After, the inoculation condition of 10⁶ cell/mL was chosen for testing wine lees 2 and 3, under the same experimental conditions. Then, MLF performance of the other two *O. oeni* strains - VP41, 450 - was also evaluated in WLM. Each strain was inoculated for a population of 10⁶ cell/mL with 0, 0.25, 0.5 g/L of wine lees 1.

Similarly, the impact of lees collected after alcoholic fermentation and after ageing was evaluated in red Merlot wine from CDO S. Emilion (France). The general chemical parameters of wine were: 12.45 % (vol/vol) of ethanol, 2.07 g/L of L-malic acid and pH 3.41. This wine had an initial LAB population of around 10³ CFU/mL. 10 mL of wine were inoculated with 1 g/hL of commercial freeze-dried *O. oeni* VF, corresponding to approximately 10⁴ and 10⁶ cell/mL, respectively. A control condition without inoculation of *O*. *oeni* was also assessed. MLF performance was evaluated in presence of 0, 0.25, and 0.5 g/L of wine lees (1, 2, and 3). Fermentations were conducted at 20 °C, and L-malic acid concentration was monitored periodically. Each condition was performed in duplicate.

### 5. Brettanomyces bruxellensis growth

The effect of wine lees in *B. bruxellensis* growth was evaluated in two wines with three different strains. 0.5 g/L of wine lees (1 and 4) were added to wine A (Petit Verdot wine from Château Couhins, 14.52 % vol/vol of ethanol, 1.75 g/L of L-malic acid, 0.6 g/L of residual sugars at pH 3.89.), and wine B-(Cabernet Franc, 14.46 % vol/vol of ethanol, 1.25 g/L of L-malic acid, 0.7 g/L of residual sugars at pH 3.71) from Château Tournefeuille. A control of each wine without addition of wine lees was used. Each wine was inoculated with each of the three *B. bruxellensis* strains (CRBO L0611, diploid from wine; CRBO-L0422, triploid from beer; CRBO L0424, triploid from wine) for a population of 10² cell/mL. Before inoculation, cells were progressively acclimated to each wine from the initial culture medium. Wines were incubated statically at 20 °C. Wine A and wine B had an initial total yeast population of 4.35 × 10³ and 3.68 × 10³ CFU/mL, respectively. Non-*Saccharomyces* yeasts were not detected by plating (< 10 CFU/mL). *B. bruxellensis* growth was evaluated at day 0, 9, 22, and 35 after inoculation by plating on non-*Saccharomyces* solid medium (20 g/L glucose, 10 g/L yeast extract, 10 g/L peptone, 10 g/L agar, chloramphenicol 100 mg/L, biphenyl 150 mg/L and cycloheximide 500 mg/L). Then, colonies suspected to be *B. bruxellensis* were confirmed by MALDI-TOF mass spectrometry. Each condition was evaluated in duplicate.

### EXAMPLE 2: RESULTS AND DISCUSSION

### 1. Analysis of lees

### 1. 1. Chemical

Wine lees were analyzed in terms of microbiological composition. Apart from *S. cerevisiae,* no other wine-related microbial species were found (data not shown). Fresh lees, before the drying process, were plated on YPD and grape juice plates. The average population on YPD was 5 × 10⁷ CFU/mL, and a MALDI-TOF identification resulted in a 100 % S. *cerevisiae* yeast population. It is not surprising since wine lees were collected from wines immediately after AF, where S. *cerevisiae* is usually the most abundant yeast species. Bacterial population found in this wine lees was lower. A total population of 1.6 × 10² CFU/mL was detected on grape juice plates, which corresponded to 100 % of *Bacillus megaterium. 8. megaterium,* and generally *Bacillus* genus, is a common environmental bacterium, typically found in the soil (Saxena et al., 2020), not related with wine production. After drying, the dominance of *S. cerevisiae* population was maintained but its concentration was reduced to 18 CFU/mg. In the case of the bacterial population found in grape juice agar, the concentration was reduced to 1.1 CFU/mg, increasing its diversity. In this step, *Streptomyces halstedii, Bacillus mycoides, Bacillus megaterium* and *Paenibacillus pabuli* were detected. All of them related with the environmental microbiome, probably due to the non-aseptic drying process.

Altogether, wine lees represent a potential microbial load when adding them to a fermentative process. Nevertheless, in the case of wine fermentation, the detected species cannot be considered as a potential risk of contamination since they are soil microorganism that do not survive in wine nor contribute to fermentatives. Besides, it is worth noting that the significant *S. cerevisiae* population detected could also contributes to AF. Still, the yeast viability in these wine lees could decrease during storage time, and specially from vintage to vintage. Interestingly, non-spoiling microorganisms were found in these lees, for instance *B. bruxellensis,* which is also related with the healthy status of the wines from whom they were collected.

### 1.2. Stimulation of lactic acid bacteria

The use of wine lees as a source of nutrients for the development of microbial biomass is one of the proposed applications of this byproduct. Several studies have used wine lees in a defined culture medium to grow different *Lactobacillus* species (Bustos et al., 2004a, 2004b) [8, 9]. These studies, using fresh lees, reported an increase in biomass production in the presence of 20 g/L of wine lees, mainly with white wine lees, and especially after distillation, where lees polyphenolic compounds are found. In our study we tested the potential use of freeze-dried wine lees as only nutrient source (apart from the 100 mL/L of grape juice) in a new culture medium. This medium was tested for the first time to grow *O. oeni.*

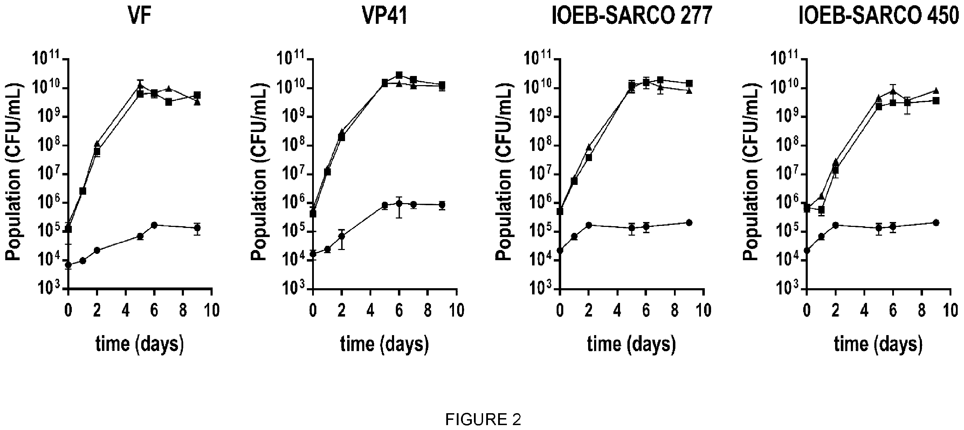

We tested two white wine lees from different origins (lees 1 and 3), and we found that the growth of four *O. oeni* strains (Figure 2) was stimulated by presence of white wine lees. Besides, no differences were observed between the growth curves obtained with the two different wine lees.

To better characterize the growth of *O. oeni* in this culture medium, we changed some parameters that directly impact the microbial growth, such as the pH and temperature. We observed that lowering the pH from 4.8 to 3.6 (a pH value of real grape must), or temperature from 25 °C to 20 °C (an easier operational setting T°), did not impact on the maximal bacterial population (data not shown). This adds value to the described culture medium to be used in low demanding conditions, as it can directly be used by diluting the grape must, adding the wine lees, and incubating at a standard room temperature, around 20 °C.

### 1.3. MLF performance

Previous works, as presented in the previous section, had demonstrated the use of wine lees to enhance LAB growth, in particular the *O. oeni* species, in culture medium. Studies in food fermentative processes are less investigated. There are few studies that evaluates the fermentative performance of sourdough (Martin-Garcia et al., 2022a) [14] or bread (Martin-Garcia et al., 2022b) [15] in presence of sparkling wine lees, and another one that investigates the effects of simulated yeast lees on MLF in synthetic wine (Balmaseda et al., 2021) [11].

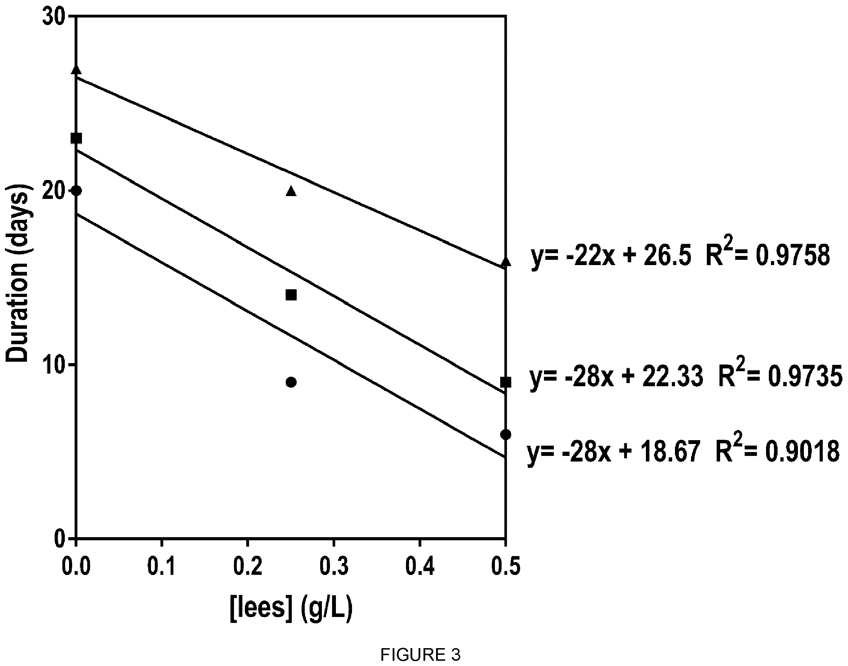

In our study we used for the first time, freeze-dried white wine lees with the purpose of enhancing MLF in wine. Using a regular inoculation regimen of 10⁶ cell/mL of *O. oeni* VF, we showed that MLF performance was ameliorated in presence of wine lees 1 with three different commercial bacterial strains (Figure 3). All strains finished MLF in presence or absence of white wine lees in the tested synthetic wine (Figure 3). In addition, we observed a linear relationship between the MLF duration, and the white wine lees concentration added in the wine: this duration is reduced by more than half in the presence of 0.5 g/L of lees (Figure 3). This was true for the three tested strain, thus, reinforcing the hypothesis of that white wine lees enhance MLF in wine conditions.

With the aim of reducing the inoculated starter culture concentration in wine with the use of wine lees, lower initial bacterial populations were tested: 10² and 10⁴ cell/mL. All these conditions were tested with *O. oeni* VF strain, again in synthetic wine with wine lees 1. Results for the MLF performance, including population and L-malic acid dynamics are tested.

Fermentations were followed for more than 100 days. It is interesting that the tendency in both, 10² and 10⁴ cell/mL was similar. There was a quick viability loss in those wines without wine lees addition, around the day 20. With the addition of 0.25 g/L of wine lees the initial inoculated bacterial population was maintained up to 60 or 120 days for 10² and 10⁴ cell/mL conditions, respectively. Still, it was not enough to reach the minimal bacterial population to perform the MLF. Finally, the addition of 0.5 g/L of wine lees, enabled a progressive bacterial growth that was enough for performing MLF (data not shown). Here, we reported that the use of wine lees at a sufficient concentration, can enable the growth of low bacterial population up to 10⁸ CFU/mL to perform MLF. In addition, these results also showed the possibility of reducing the quantity of starter culture needed to inoculate a wine. This is a promising application, still to be exploited, since the duration of the fermentation is largely extended, even if it finally concludes.

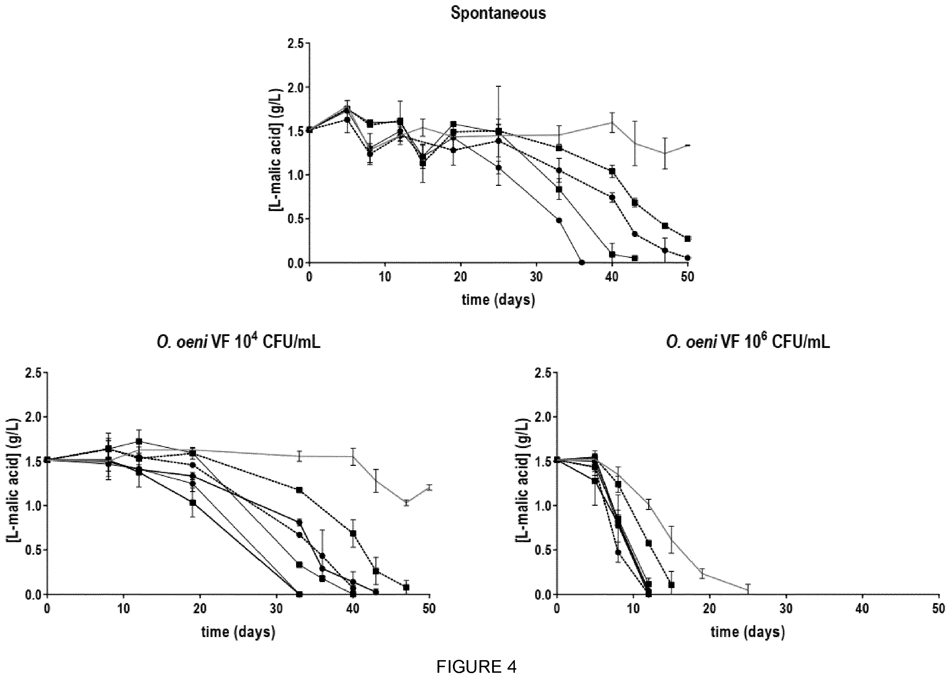

Apart from testing the effect of wine lees addition in synthetic wine, fermentations in natural red wine were also evaluated (Figure 4). Similarly to the previous tests, this wine was supplemented (or not) with 0.25 g/L and 0.5 g/L of wine lees 1, 2, and 3. These wines were inoculated with *O. oeni* VF in their commercial freeze-dried form according to manufacturer's instructions to reach a final concentration of around 10⁴ cell/mL and 10⁶ cell/mL. Also, a wine with no inoculation was followed.

As previously observed in synthetic wine (Figure 3), the addition of white wine lees to red wine enhanced MLF performance (Figure 4). First, in the wine inoculated with 10⁶ cell/mL, all conditions finished, including the control fermentation with no addition of wine lees. Generally, all the three wine lees and the two different concentrations, finished after 12 days. Besides, it was observed that wine lees 2 at 0.25 g/L lasted 15 days, a bit more than the other ones (Figure 4).

Then, those wines inoculated with 10⁴ cell/mL of *O. oeni* showed a similar tendency. Wines with no addition of wine lees did not perform MLF. In these wines, L-malic acid concentration was stuck at 1.5 g/L, which was the initial value (Figure 4). Interestingly, the positive effect on the reduction of MLF, was observed with all white wine lees, in the two inoculation conditions. This supports the idea that the observed effect is dose dependent and relays on the added white wine lees quantity, as observed in synthetic wine (Figure 3).

Moreover, considering the origin of wine lees 1 and 2, which came from the same wine but collected with three months of difference (after alcoholic fermentation/before ageing, and after ageing), we can observe interesting results (Figure 4). The results confirm previous observations concerning the stimulation of MLF by lees, with a dose-dependent effect, and they show that the lees collected after alcoholic fermentation (and before ageing/autolysis) are the most effective. Indeed, when performing MLF with no inoculation or 10⁴ cell/mL in presence of 0.5 g/L of wine lees, the duration observed is higher in wine lees 2 (43 days in spontaneous, 40 for the inoculated one), regarding to wine lees 1 (33 days in spontaneous, 36 for the inoculated one). In addition, the modality of 0.25 g/L of lees 2 did not enable MLF completion. This points that during ageing, the quality of the recovered wine lees changes. In this sense, the observed results could be related with changes in the nutritional properties of wine lees when using them as MLF potentiator. Therefore, the winemaking process impact quality of lees and is important for maximum efficiency in MLF.

Overall, the results observed with these wine lees enhanced *O. oeni* growth and MLF performance in synthetic and natural wine. In addition, it enabled the completion of MLF in wines with low bacterial population, which did not finish in the case of no addition of wine lees. We also observed that the drying process did not interfere with the positive effect here observed. Freeze-drying could represent a suitable tool for preserving and storing wine lees and enables an easier dosage comparing to fresh lees. The added concentration of wine lees in this study were 0.25 g/L and 0.5 g/L, which corresponds to 25 g/hL and 50 g/hL, respectively. These concentrations are not unfamiliar to other oenological preparations (also commercialized in powder form) used as activators for AF or MLF, which also range from 20 to 50 g/hL. That is why the use and concentrations proposed in this study are in form for an easy industrial adaptation.

### 1.4. Brettanomyces bruxellensis

After studying the positive effect of wine lees in *O. oeni* growth, we wanted to test if the addition of wine lees in wine had also a growth promoting effect in a very well-known spoiling microorganism as *B. bruxellensis. B. bruxellensis* growth in wine can compromise the quality of the product due to the production of volatile phenols. In this sense, it was important to address the effect of wine lees addition in *B. bruxellensis* growth. There is not much literature addressing the effects of wine lees on *B. bruxellensis.* However, it is already described that red wine lees contains high microbial population during vinification, and usually *B. bruxellensis* is mostly detected in these red wine lees (Renouf and Lonvaud-Funel, 2007) [16-17].

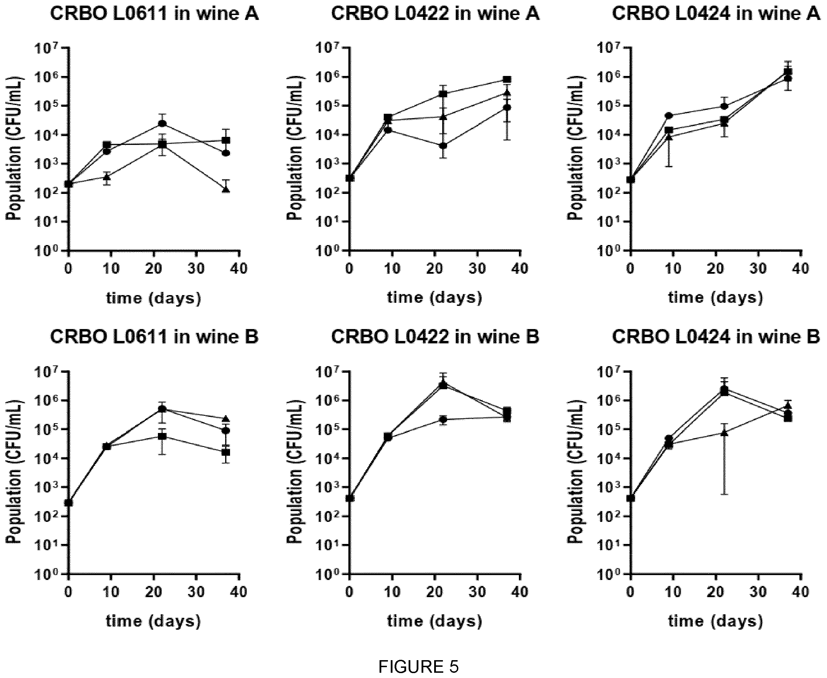

In this test we did not observe that the addition of wine lees promoted *B. bruxellensis* growth in red wine (Figure 5). Three *B. bruxellensis* strains were inoculated at an approximately 10²- 10³ CFU/mL in two different wines. At the same time, wines were supplemented (or not) with lees 1 and 4. Viability of the yeast was monitored for 40 days.

In general, the addition of wine lees at the tested concentration of 0.5 g/L did not significantly promote the growth of the yeast in the tested conditions. However, it was observed a contrary tendency for CRBO L0422 at day 21, where the two wines with wine lees presented higher population than the non-supplemented one (Figure 5). But this difference was significantly reduced by day 40, to values like the non-supplemented wines. The addition of wine lees did not stimulate the development of contamination yeasts. This result, confirmed on several types of wine, suggests that the lees are a necessary nutrient for lactic acid bacteria, but dispensable for other micro-organisms such as *B. bruxellensis* yeast. It also suggests that the use of lees does not increase the risk of development of this yeast and the appearance of the defects it causes.

A chemical analysis of the wine after MLF was carried out. The addition of lees does not seem to affect the wine's classic oenological parameters (pH, total acidity, total polyphenols). A general analysis of the aromatic esters contributing to the wine's fruity aroma was also carried out. No reduction in this family of molecules was observed. Professional tasting of the treated wines did not reveal any negative impact of the lees on the treated wine after MLF (data not shown).

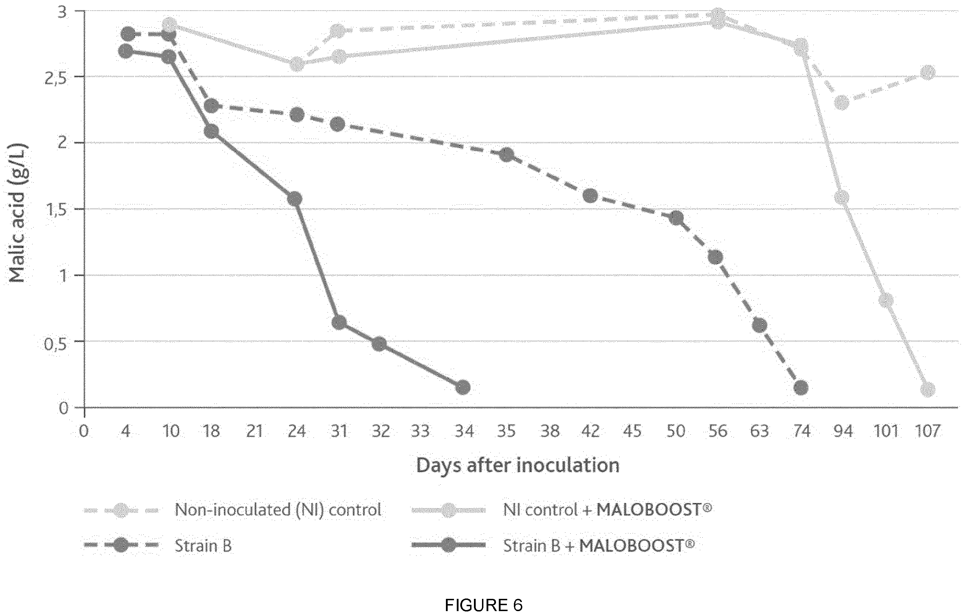

Taken together, these results are promising for the use of lees to improve MLF in wines. Our results are not only comparable to those obtained with commercial products, *e.g.* Biolaffort's Maloboost^{®} product, but also better (faster MLF kinetics) (Figure 6). It is also to be noted that Biolaffort's Maloboost^{®} product improves MLF only in the case of lactic bacteria seeding and at doses of 20-40 g/hL.

### 2. Conclusion

The potential exploitation of white wine lees (after alcoholic fermentation/before ageing) in wine industry, particularly with the aim of enhancing red wine MLF, was positively tested.

First, the characterization of wine lees brough a general but useful data according to their main compounds. Using the lees in dry form helps to preserve them and ensures that there is no microbiological contamination.

Second, industrializing the process of recycling white wine lees not only reduces the wine industry's carbon footprint, but also produces a low-cost culture medium based on diluted grape juice and wine lees tested and proved as suitable for producing wine related LAB, commonly used for performing MLF, as *O. oeni.* It was sufficient a medium containing 100 mL/L of grape juice and 10 g/L of freeze-dried wine lees to produce up to 10¹⁰ CFU/mL.

Third, it was demonstrated that the addition of white wine lees can reduce MLF duration in high bacterial population wines, and at the same time, enable bacterial growth up to the limit to perform the MLF, and thus, be able to conclude the fermentation without the need to inoculate with lactic acid bacteria before MLF.

Fourth, the addition of white wine lees in red wine does not increase or not significantly the growth of spoilage microorganisms as *B. bruxellensis.*

Altogether, this work an integrative study in which white wine lees are proposed as LAB growth activator, and it is demonstrated that there is no potential microbial spoilage risk or that it could compromise the aromatic quality of wine. It also opens up the possibility of using white wine lees in other areas where it may be useful to promote the growth of lactic acid bacteria (fermented products or industrial production).

### List of references

[1] Devesa-Rey et al., Environ. Monit. Assess., 179(1-4): 371-388, 2011
[2] Ruiz-Moreno M. J et al., Industrial Crops and Products, 63: 152-157, doi.org/10.1016/j.indcrop.2014.10.016, 2015
[3] Yammine S., et al., Oeno One, 53(3). doi.org/10.20870/.oeno-one.2019.53.3.2343, 2019
[4] De Iseppi et al., Food Research International, 137: 109352. doi: 10.1016/j.foodres.2020.109352, 2020
[5] De Iseppi et al., LWT, Food Science and Technology, 136: 112273. doi.org/10.1016/j.lwt.2021.112273, 2021
[6] Nioi, Congress AFSEP, ISVV, May 30 2022, Bordeaux
[7] Fornairon-Bonnefond, C. et al., Journal International des Sciences de la Vigne et du Vin, 35(2) : 57-78, 2001
[8] Bustos et al., Journal of Agricultural and Food Chemistry, 52(16) : 5233-5239, 2004a
[9] Bustos et al., Journal of Agricultural and Food Chemistry, 52(4): 801-808, 2004b
[10] Balmaseda et al., Foods, 10(7): 1540, doi: 10.3390/foods10071540, 2021a
[11] Balmaseda et al., Food Microbiology, 99: 103839. doi.org/10.1016/j.fm.2021.103839, 2021b
[12] Balmaseda et al., Int. J. Food Microbiol., 362: 109490, doi: 10.1016/j.ijfoodmicro.2021.109490, 2022
[13] Balmaseda et al., Int. J. Food Microbiol., 400: 110276, doi: 10.1016/j.ijfoodmicro.2023.110276, 2023
[14] Martin-Garcia et al., Foods, 11(9) : 1-13, 2022a
[16] Martin-Garcia, Fermentation, 8(3) : 4-13, 2022b
[16] Renouf et Lonvaud-Funel, Microbiol. Res., 162(2) : 154-167, 2007
[17] Paulin at al., Front. Microbiol., 11:571067, 2020

## Claims

1. Method of inducing in a food product the conversion of L-malic acid to L-lactic acid and/or growth of lactic acid bacteria comprising the following steps:
f) recovery of white wine lees after alcoholic fermentation and before wine ageing;
g) drying of white wine lees from step a);
h) addition of dried white wine lees from step b) to the food product;
i) keeping of the food product from step c) under conditions which allow conversion of malic acid to lactic acid;
j) recovery of the malolactically fermented food product.

2. Method according to claim 1, wherein the drying in step b) is carried out by atomisation or freeze-drying.

3. Method according to claim 2, wherein the dried white wine lees contain from 90 % to 95 % of dry matter.

4. Method according to any one of claims 1 to 3, wherein the addition in step c) is carried out at a concentration of 0.1 to 1 g/L of dried white wine lees.

5. Use of dried white wine lees to promote malolactic fermentation in a food product.

6. Method according to any one of claims 1 to 4 or use according to claim 5, wherein the food product is chosen from the group consisting of fermented alcoholic drinks, fruit juices, dairy products, fermented vegetables, cold meats, sourdough bread.

7. Method or use according to claim 6, wherein the fermented alcoholic drinks are chosen from the group consisting of red wines, beers and ciders.

8. Powdered nutrient comprising dried white wine lees.
